# EUROPEAN PATENT APPLICATION

(11) **EP 2 239 555 A1**
(43) Date of publication of application: **13.10.2010**
(21) Application number: 08862219.6
(22) Date of filing: 15.12.2008
(51) Int. Cl.: G01N 1/36, C12N 15/09, C12Q 1/68, G01N 1/28, G01N 33/48, G01N 33/53

(54) **NOVEL METHOD OF PREPARING SPECIMEN HAVING EXCELLENT ABILITIES TO MAINTAIN TISSUE MORPHOLOGY AND MAINTAIN NUCLEIC ACID QUALITIES**

(30) Priority: 14.12.2007 JP 2007322944
(71) Applicant: Chugai Seiyaku Kabushiki Kaisha, Kita-ku Tokyo 115-8543 (JP); Pharmalogicals Research Pte. Ltd., Helios 138667 (SG)
(72) Inventor: WATANABE, Takeshi, Helios 138667 (SG); YANTING, Fang, Helios 138667 (SG); MATSUBARA, Kouichi, Helios 138667 (SG); SUZUKI, Masami, Gotenba-shi Shizuoka 412-8513 (JP); TERASHIMA, Hiromichi, Kamakura-shi Kanagawa 247-8530 (JP); MIZUNO, Hideaki, Kamakura-shi Kanagawa 247-8530 (JP)
(74) Representative: Vossius & Partner
(86) International application number: PCT/JP2008/072787
(87) International publication number: WO 2009/078386

(57) **Abstract**

The present invention aims to develop a method for specimen preparation, which ensures the maintenance of both tissue morphology and nucleic acid quality (particularly RNA quality). The present invention further aims to prepare a specimen by this method, from which desired cells are then collected by microdissection and analyzed for gene expression.

A method for specimen preparation from Various frozen or unfrozen organs or tissues (excluding hard tissues) of the whole body, which comprises the following steps:
1) fixing a target organ or tissue with PFA fixative; and
2) embedding the same in paraffin by the AMeX method.

SEQUENCE LISTING

<110> PHARMALOGICALS RESEARCH PTE. LTD.
      CHUGAI SEIYAKU KABUSHIKI KAISHA

<120> NOVEL METHOD FOR SPECIMEN PREPARATION, WHICH ENSURES PRESERVATION
      OF TISSUE MORPHOLOGY AND NUCLEIC ACID QUALITY

<130> S2112 EP S3

<140> EP 08 86 2219.6
<141> 2008-12-15

<150> JP 2007-322944
<151> 2007-12-14

<160> 5

<170> PatentIn version 3.1

<210> 1
<211> 1761
<212> DNA
<213> Homo sapiens

<400> 1
ttgccgatcc gccgcccgtc cacacccgcc gccagctcac catggatgat gatatcgccg 60

cgctcgtcgt cgacaacggc tccggcatgt gcaaggccgg cttcgcgggc gacgatgccc 120

cccgggccgt cttcccctcc atcgtggggc gccccaggca ccagggcgtg atggtgggca 180

tgggtcagaa ggattcctat gtgggcgacg aggcccagag caagagaggc atcctcaccc 240

tgaagtaccc catcgagcac ggcatcgtca ccaactggga cgacatggag aaaatctggc 300

accacacctt ctacaatgag ctgcgtgtgg ctcccgagga gcaccccgtg ctgctgaccg 360

aggcccccct gaaccccaag gccaaccgcg agaagatgac ccagatcatg tttgagacct 420

tcaacacccc agccatgtac gttgctatcc aggctgtgct atccctgtac gcctctggcc 480

gtaccactgg catcgtgatg gactccggtg acggggtcac ccacactgtg cccatctacg 540

aggggtatgc cctcccccat gccatcctgc gtctggacct ggctggccgg gacctgactg 600

actacctcat gaagatcctc accgagcgcg gctacagctt caccaccacg gccgagcggg 660

aaatcgtgcg tgacattaag gagaagctgt gctacgtcgc cctggacttc gagcaagaga 720

tggccacggc tgcttccagc tcctccctgg agaagagcta cgagctgcct gacggccagg 780

tcatcaccat tggcaatgag cggttccgct gccctgaggc actcttccag ccttccttcc 840

tgggcatgga gtcctgtggc atccacgaaa ctaccttcaa ctccatcatg aagtgtgacg 900

tggacatccg caaagacctg tacgccaaca cagtgctgtc tggcggcacc accatgtacc 960

ctggcattgc cgacaggatg cagaaggaga tcactgccct ggcacccagc acaatgaaga 1020

tcaagatcat tgctcctcct gagcgcaagt actccgtgtg gatcggcggc tccatcctgg 1080

cctcgctgtc caccttccag cagatgtgga tcagcaagca ggagtatgac gagtccggcc 1140

cctccatcgt ccaccgcaaa tgcttctagg cggactatga cttagttgcg ttacaccctt 1200

tcttgacaaa acctaacttg cgcagaaaac aagatgagat tggcatggct ttatttgttt 1260

tttttgtttt gttttggttt tttttttttt tttggcttga ctcaggattt aaaaactgga 1320

acggtgaagg tgacagcagt cggttggagc gagcatcccc caaagttcac aatgtggccg 1380

aggactttga ttgcacattg ttgttttttt aatagtcatt ccaaatatga gatgcattgt 1440

tacaggaagt cccttgccat cctaaaagcc accccacttc tctctaagga gaatggccca 1500

gtcctctccc aagtccacac aggggaggtg atagcattgc tttcgtgtaa attatgtaat 1560

gcaaaatttt tttaatcttc gccttaatac ttttttattt tgttttattt tgaatgatga 1620

gccttcgtgc ccccccttcc ccctttttgt cccccaactt gagatgtatg aaggcttttg 1680

gtctccctgg gagtgggtgg aggcagccag ggcttacctg tacactgact tgagaccagt 1740

tgaataaaag tgcacacctt a 1761

<210> 2
<211> 20
<212> DNA
<213> Artificial sequence

<220>
<221> source
<223> /note= "Description of artificial sequence: Primer for amplifying
the 5' side of human beta-actin."

<400> 2
acaatgtggc cgaggacttt 20

<210> 3
<211> 20
<212> DNA
<213> Artificial sequence

<220>
<221> source
<223> /note= "Description of artificial sequence: Primer for amplifying
the 5' side of human beta-actin."

<400> 3
tgtgtggact tgggagagga 20

<210> 4
<211> 29
<212> DNA
<213> Artificial sequence

<220>
<221> source
<223> /note= "Description of artificial sequence: Primer for amplifying
the 3' side of human beta-actin."

<400> 4

ttgttttatt ttgaatgatg agccttcgt 29

<210> 5
<211> 25
<212> DNA
<213> Artificial sequence

<220>
<221> source
<223> /note= "Description of artificial sequence: Primer for amplifying
the 3' side of human beta-actin."

<400> 5
ggtgtgcact tttattcaac tggtc 25

## Description

### TECHNICAL FIELD

The present invention relates to a method for tissue specimen preparation, which ensures the maintenance of tissue morphology and nucleic acid quality. More specifically, the present invention relates to a method for tissue specimen preparation, which is based on a combination of fixation with PFA fixative and embedding in paraffin by the AMeX method. The present invention also relates to a tissue specimen obtainable by the above method, a method for testing the quality of mRNA in cells within such a tissue specimen, and a method for preparing a sample suitable for DNA microarray gene expression analysis by using such a tissue specimen.

### BACKGROUND ART

When clinical samples or laboratory animal samples are used in gene expression analysis by Gene Chip (DNA microarray) technology, nucleic acids have usually been extracted from tissue grafts of a certain size and used for analysis. However, tissue grafts contain various cells other than target cells, and hence these various cells have also been included for analysis in conventional cases. To solve this problem, a technique has been developed, in which specific cells to be targeted are collected from thin sections prepared by histopathological procedures (microdissection) and used for analysis. In recent years, instruments used for this purpose have been commercially available from a plurality of manufacturers and used widely.

For microdissection, thin sections prepared from frozen tissue samples embedded in OCT compound or the like are generally used. Thin sections of frozen tissue samples (frozen sections) are obtained simply by freezing fresh tissue grafts, and such simple preparation ensures the maintenance of nucleic acid quality. However, in frozen sections, tissue morphology cannot be maintained satisfactorily, which often makes it difficult to sufficiently distinguish cells from one another, depending on the type of tissue to be used or cell to be targeted. For example, in the case of prostate cancer tissues containing normal region, hyperplastic region, precancerous region (PIN; prostatic intraepithelial neoplasia) and cancer region in combination, it has been very difficult to distinguish and isolate only cancer cells. On the other hand, formalin-fixed paraffin sections conventionally prepared ensure good maintenance of tissue morphology, but suffer from serious degradation and degeneration of nucleic acids during fixation and processing, which has made it impossible to carry out an accurate gene expression analysis with nucleic acids (RNAs) of good quality.

Since tissue fixation also corresponds to denaturation of proteins or nucleic acids, the maintenance of tissue morphology and the avoidance of nucleic acid degradation are, so to speak, problems that are mutually contradictory. As a result of many years of studies, the inventors have solved the mutually contradictory problems, i.e., the maintenance of tissue morphology and the maintenance of antigen proteins, by development of the PLP-AMeX method (Patent Document 1, Non-patent Document 1, Non-patent Document 2). PLP (periodate-lysine-paraformaidehyde) fixative has been developed for preparation of frozen sections that are intended for use in immunohistochemical staining, with the aim of minimizing the influence on immune reactions and maintaining tissue morphology (Non-patent Document 3, Non-patent Document 4, Non-patent Document 5). The PLP fixative refers to a fixative composed of periodate-lysine-paraformaldehyde, and its general composition is as follows: 0.01 M NaIO₄, 0.075 M lysine, 0.0375 M phosphate buffer, and 2% paraformaldehyde. The paraformaldehyde concentration may be adjusted as appropriate, generally in the range of 1% to 6 %. On the other hand, the AMeX method (acetone, methyl benzoate and xylene method) is a relatively new paraffin-embedding method developed by Sato et al. (Non-patent Document 6, Non-patent Document 7), which is reported to allow detection of even antigens that are difficult to detect in ordinary paraffin sections.
Patent Document 1: Japanese Patent Public Disclosure No. 2002-82026
Nora-pater Document 1: Suzuki M et. al., Histol Histopathol 1999; 46: 679-86
Non-patent Document 2: Suzuki M et al., Exp Anim 1998; 47: 211-14
Non-patent Document 3: McLean IW et al., J Histochem Cytochem 1974; 22: 1077-83
Non-patent Document 4: Sisson Sp et al., J Histochem Cytochem 1980; 28: 441-52
Non-patent Document 5: Rantala I et al., J Histochem Cytochem 1982; 30: 932-37
Non-patent Document 6: Sato Y et al., Am J Pathol 1986; 125: 431-35
Non-patent Document 7: Sato Y et al., Am J Pathol 1992; 140: 775-79

### DISCLOSURE OF THE INVENTION

### PROBLEMS TO BE SOLVED BY THE INVENTION

In the above methods conventionally used for specimen preparation, it was difficult to ensure the maintenance of both tissue morphology and nucleic acid quality. An object of the present invention is to develop a method for specimen preparation, which ensures the maintenance of both tissue morphology and nucleic acid quality (particularly RNA quality). Another object of the present invention is to prepare a specimen by this method, from which desired cells are then collected by microdissection and analyzed for gene expression.

### MEANS FOR SOLVING THE PROBLEMS

As a result of extensive and intensive efforts made to achieve the above objects, the inventors of the present invention have found that the combined use of fixation with PFA (paraformaldehyde) and embedding in paraffin by the AMeX method allows preparation of specimens that ensure the maintenance of tissue morphology and nucleic acid quality (particularly RNA quality).

The inventors of the present invention have further developed a technique of great significance in using tissues from tissue banks. In standard tissue banks, most tissue samples are frozen for storage (SNAP-Frozen samples), and these samples have been used primarily for extraction of nucleic acids and/or proteins. Although it is generally possible to use SNAP-Frozen samples for preparation of frozen sections, tissue morphology cannot be maintained satisfactorily in these sections when compared to ordinary paraffin sections, which often makes it difficult to sufficiently distinguish cells from one another. The inventors of the present invention have also found that when SNAP-Frozen samples are processed by the PFA-AMeX-Paraffin method, it is possible to prepare paraffin blocks that ensure the maintenance of tissue morphology without impairing nucleic acid quality (particularly RNA quality).

Namely, the present invention provides the following.
(1) A method for specimen preparation from various frozen or unfrozen organs or tissues (excluding hard tissues) of the whole body, which comprises the following steps:
   1) fixing a target organ or tissue with PFA fixative; and
   2) embedding the same in paraffin by the AMeX method.
(2) The method according to (1) above, wherein fixation with the PFA fixative is accomplished by immersion.
(3) The method according to (1) or (2) above, which further comprises the step of preparing thin sections after paraffin embedding by the AMeX method.
(4) The method according to (3) above, which further comprises the step of deparaffinizing and rehydrating the resulting thin sections.
(5) The method according to (4) above, which further comprises the step of tissue staining, immunohistochemical staining or enzyme histochemical staining.
(6) The method according to any one of (1) to (5) above, which is a method for preparing a prostate tissue specimen.
(7) The method according to any one of (1) to (5) above, which is a method for preparing a prostate cancer tissue specimen.
(8) Specimens of various organs or tissues (excluding hard tissues) of the whole body, which are obtainable by the method according to any one of (1) to (5) above.
(9) The specimen according to (8) above, which is a prostate tissue specimen.
(10) The specimen according to (8) above, which is a prostate cancer tissue specimen.
(11) A method for testing the quality of mRNA in cells within an organ or tissue specimen, which comprises the following steps:
   1) collecting desired cells from an organ or tissue specimen obtainable by the method according to any one of (1) to (7) above;
   2) extracting total RNA from the collected desired cells;
   3) synthesizing cDNA from the extracted total RNA by reverse transcription reaction;
   4) using the synthesized DNA as a template to amplify the 5- and 3-terminal regions of β-actin by PCR; and
   5) calculating the ratio of amplification products between 5- and 3-terminal regions of β-actin, whereby cells whose 3-terminal region/5-terminal region PCR product ratio is below a given value are determined as having good mRNA quality.
(12) The method according to (11) above, wherein the desired cells are collected by microdissection from the organ or tissue specimen.
(13) A method for preparing a DNA microarray sample, which comprises the following steps:
   1) collecting desired cells from an organ or tissue specimen obtainable by the method according to any one of (1) to (7) above;
   2) testing the quality of mRNA in the collected desired cells in the following manner:
      a) extracting total RNA from the collected desired cells;
      b) synthesizing cDNA from the extracted total RNA by reverse transcription reaction;
      c) using the synthesized cDNA as a template to amplify the 5- and 3-terminal regions of β-actin by PCR; and
      d) calculating the ratio of amplification products between 5- and 3-terminal regions of β-actin, whereby cells whose 3-terminal region/5-terminal region PCR product ratio is below a given value are determined as having good mRNA quality; and
   3) extracting total RNA from the cells determined in step 2) as having good mRNA quality and using the same to synthesize cDNA, from which cRNA is further synthesized.
(14) A method for performing hierarchical clustering analysis in a DNA microarray, which uses the sample obtained by the method according to (13) above.

### ADVANTAGES OF THE INVENTION

The PFA-AMeX-Paraffin method, in which fixation with PFA is combined with embedding in paraffin by the AMeX method, is a method for specimen preparation which ensures the maintenance of both tissue morphology and nucleic acid quality. In the case of tissues (e.g., prostate cancer tissue) whose cells are difficult to sufficiently distinguish in frozen tissue sections, this method is found to be very useful in collecting target cells by microdissection under observation of tissue morphology, and further in analyzing gene expression in these cells by using a DNA microarray.

The inventors of the present invention have further found that when samples frozen for storage (SNAP-Frozen samples) are processed by the PFA-AMeX-Paraffin method, it is possible to prepare paraffin blocks that ensure the maintenance of tissue morphology without impairing nucleic acid quality. This finding indicates that the PFA-AMeX-Paraffin method is very useful in preparing paraffin blocks that ensure the maintenance of tissue morphology without impairing nucleic acid quality, starting from frozen tissues in standard tissue banks (tissue samples are generally frozen for storage).

### BRIEF DESCRIPTION OF THE DRAWINGS

[Figure 1] Figure 1 is a photograph under a microscope showing a HE-stained tissue image of a thin section from a clinical frozen tissue specimen of human prostate cancer tissue.
[Figure 2] Figure 2 is a photograph under a microscope showing a HE-stained tissue image of a thin section from a clinical PFA-AMeX-Paraffin specimen of human prostate cancer tissue.
[Figure 3] Figure 3 shows the results of hierarchical clustering analysis of gene expression profiles in prostate cancer and prostatic hyperplasia tissues, along with the results of tissue morphological observation of each specimen. In the tissue morphological observation, determination was made according to the criteria described in the General Rule for Clinical and Pathological Studies on Prostate Cancer, third edition (edited by Japanese Urological Association and Japanese Society of Pathology, Kanehara Publishing, 2001).
   In the dendrogram, 128, 112, 133 and 140, which were samples from prostate patient tissues, were found to have prostatic hyperplasia, as analyzed by tissue morphological observation.
[Figure 4] Figure 4 is a photograph under a microscope showing a HE-stained tissue image of a thin section from a specimen prepared from SNAP-Frozen (frozen) human prostate cancer tissue by the PFA-AMeX-Paraffin method.

### BEST MODE FOR CARRYING OUT THE INVENTION

The specimen preparation method of the present invention is applicable to a wide range of animals, regardless of their type, including amphibians, reptiles, birds and mammals, with mammals being particularly preferred.

As used herein, the phrase "various organs or tissues (excluding hard tissues) of the whole body" is intended to mean all organs or tissues distributed over the whole body of the above animals, except for hard tissues such as bone tissue. An organ or tissue particularly suitable for the method of the present invention is prostate tissue.

The method of the present invention can be used for frozen or unfrozen organs or tissues. Thus, tissue samples frozen for storage (e.g., SMAP-Frozen samples), which are obtained from tissue banks, also fall within the present invention.

In the method of the present invention, an organ or tissue excised from an animal is first fixed with PFA fixative. The term "PFA fixative" refers to a solution for cell fixation, prepared by diluting a 1% to 6% aqueous paraformaldehyde solution with a buffer (e.g., phosphate buffer). Preferably, 4% PFA fixative (4% paraformaldehyde/0.01 M PBS (pH 7.4)) is used for this purpose.

Fixation with PFA fixative in the present invention may be accomplished by immersing a target organ or tissue in PFA fixative containing 1% to 6% (preferably 4%) paraformaldehyde at a temperature of 0°C to 8°C, preferably about 4°C for 2 to 40 hours, preferably 6 to 30 hours.

Then, the fixed organ or tissue is washed with phosphate-buffered saline or the like. In this case, a part desired to be observed may be excised from the organ or tissue before being washed.

The organ or tissue thus prepared is then embedded in paraffin by the AMeX method. The AMeX method is a paraffin-embedding method involving fixation in cold acetone, dehydration with acetone, clearing with methyl benzoate and xylene, and embedding in paraffin. More specifically, the organ or tissue is immersed in acetone at -25°C to 8°C, preferably -20°C to 6°C for 2 to 24 hours, preferably 4 to 16 hours. Next, the acetone containing the tissue is warmed to room temperature, or alternatively, the organ or tissue is transferred to another acetone kept at room temperature, followed by dehydration at room temperature for 0.5 to 5 hours, preferably 1 to 4 hours. Next, the organ or tissue is cleared by being immersed in methyl benzoate at room temperature for 0.5 to 3 hours, preferably 0.5 to 2 hours and in xylene at room temperature for 0.5 to 3 hours, preferably 0.5 to 2 hours, and then embedded by being soaked in paraffin at 55°C to 65°C, preferably 58°C to 62°C for 1 to 4 hours, preferably 1 to 3 hours. The organ or tissue paraffin block thus obtained by the PFA-AMeX method is stored at low temperature until use.

At the time of use, the paraffin block obtained above is sliced with a microtome or the like to prepare thin sections, followed by deparaffinization and rehydration of the thin sections. Deparaffinization and rehydration may be accomplished in a known manner.
For example, deparaffinization may be accomplished with the use of xylene and/or toluene, while rehydration may be accomplished with the use of alcohol and/or acetone.

The thin sections thus obtained are optionally further stained by tissue staining, immunohistochemical staining or enzyme histochemical staining, and then provided for observation.

When specimens prepared by the method of the present invention are stained by tissue staining (special staining), any type of staining available for use in ordinary paraffin-embedded sections can be used (e.g., PAS staining, Giemsa staining, toluidine blue staining). Moreover, all antibodies suitable for immunohistochemical staining can be used (e.g., various antibodies against cell surface antigens, cytoskeletons, extracellular matrices, cytokines, adhesive molecules and so on). For enzyme histochemical staining, stains available for use on sections can be used (e.g., various stains including ALP, ACP, TRAP, and esterase). To stain pathological tissues, examples of staining available for use include Hematoxylin-Eosin staining for general purposes; Van Gieson staining, Azan staining, and Masson Trichrome staining for collagen fibers; Weigert staining and Elastica Van Gieson staining for elastic fibers; and Watanabe's silver impregnation staining and PAM staining (Periodic acid methenamine silver stain) for reticular fibers and basement membranes.

The present invention also provides specimens of various organs or tissues (excluding hard tissues) of the whole body, which are obtainable by the above method. Particularly preferred specimens are those of prostate tissue and prostate cancer tissue, but are not limited thereto.

Organ or tissue specimens obtainable by the method of the present invention can be used for quantification of total RNA and test for mRNA quality in cells within the organ or tissue specimens. To collect desired cells from the tissue specimens, it is preferable to use microdissection techniques, particularly laser microdissection (LMD). LMD is a new research tool which uses an instrument having a laser irradiator connected to a microscope, by which a clump of target cells on a tissue section can be excised, picked and collected by the laser while monitoring the section under a microscope. Since this technique allows collection of a target cell group from a body tissue, it is possible to precisely know how much a specific gene is expressed in which of various cells constituting the tissue in the body. As an instrument for microdissection, for example, an AS-LMD system (Leica Microsystems) may be used.

Then, the collected desired cells may be treated in a known manner to extract and quantify total RNA. Moreover, after RNA quantification, mRNA is reverse-transcribed with oligo dT primers and reverse transcriptase to synthesize cDNA. The synthesized cDNA is used as a template to amplify the 5- and 3-terminal regions of β-actin by PCR, followed by calculating the ratio of amplification products between 5- and 3-terminal regions of β-actin, whereby total RNA can be tested for degradation or mRNA can be tested for its quality. Since β-actin is a gene expressed in abundance in all tissues and cells, it can be used as an indicator for quality comparison of RNAs contained in various tissues and cells. In the present invention, samples whose 3-terminal region/5-terminal region PCR product ratio is below a given value are determined as having good RNA quality. The 3-terminal region/5-terminal region PCR product ratio is preferably 20 or less, more preferably 10 or less, and even more preferably 5 or less.

In the present invention, a sample determined as having good mRNA quality by the above method may further be used to prepare a DNA microarray sample. From the total RNA of such a sample determined as having good mRNA quality, a labeled cRNA probe may be prepared, for example, according to standard procedures provided by Affymetrix, Inc., and gene expression data may be obtained using a DNA microarray and provided for clustering analysis.

For example, in hierarchical clustering (hierarchical cluster analysis technique), data are hierarchically classified on the basis of similarity, and the results obtained can be expressed as a tree diagram. The use of this technique makes it easy to understand the implicational relationship between clusters in a wide range from detailed classification to rough classification. As shown in the Example section described later, in the case of using cells obtained from specimens prepared by the PFA-AMeX-Paraffin method, prostate cancer specimens and prostatic hyperplasia specimens showed mutually different gene expression patterns, so that the prostate cancer specimen group and the prostatic hyperplasia specimen group were distinguished as separate clusters. On the other hand, there was a high similarity in gene expression pattern between prostate cancer specimens or between prostatic hyperplasia specimens. Further, among the prostate cancer specimens, those determined by morphological observation as being poorly-differentiated adenocarcinoma or moderately-differentiated adenocarcinoma were found to show a higher similarity in gene expression pattern between poorly-differentiated adenocarcinoma specimens or between moderately-differentiated adenocarcinoma specimens than between poorly-differentiated and moderately-differentiated adenocarcinoma specimens, and specimens determined by morphological observation as being poorly-differentiated were found to form a cluster earlier than others. This result indicated that gene expression patterns faithfully reflecting the results of morphological observation were obtained.

In standard tissue banks, most tissue samples are frozen for storage (SNAP-Frozen samples), and these samples have been used primarily for extraction of nucleic acids and/or proteins. As to tissue morphological observation, although it is generally possible to use SNAP-Frozen samples for preparation of frozen sections, they have a difficulty in the maintenance of tissue morphology when compared to ordinary paraffin sections. For this reason, specimens were prepared for SNAP-Frozen samples (frozen tissues) by the PFA-AMeX-Paraffin method, and these specimens were evaluated for the maintenance of tissue morphology and for mRNA quality. As a result, in thin paraffin sections of the specimens prepared from the SNAP-Frozen (frozen) samples by the PFA-AMeX-Paraffin method, it was possible to distinguish tissue morphology with substantially the same accuracy as in thin paraffin sections of PFA-AMeX-Paraffin specimens prepared from unfrozen tissues, while the mRNAs maintained were confirmed to be of substantially the same quality as that of specimens prepared from unfrozen samples.

### EXAMPLES

The present invention will be further described in more detail by way of the following examples, which are not intended to limit the invention. Various changes and modifications can be made by those skilled in the art, and these changes and modifications also fall within the present invention.

It should be noted that for all of the specimens used in the following examples, IC (Informed Consent) was obtained from patients and their consent was also obtained for use of the specimens for research purposes.

### Example 1: Preparation of specimens

### (1) Preparation of specimens by PLP-AMeX-Paraffin method or PFA-AMeX-Paraffin method and observation of tissue morphology

The tissues used were obtained from patients with prostate cancer or benign prostatic hyperplasia who consented to use of their excised tissues for research purposes. The obtained samples were each trimmed to about 5 mm³ size and fixed in PLP fixative (0.01 M periodate, 0.075 M lysine, 0.0375 M phosphate buffer, 4% paraformaldehyde) or in 4% PFA fixative (4% paraformaidehyde/0.01 M PBS (pH 7.4)) at 4°C for 16 to 24 hours. The fixed samples were washed with a 0.01 M PBS solution (at 4°C for 2 hours) and then dehydrated with acetone (at 4°C overnight and at room temperature for 30 minutes × 4), followed by treatment with methyl benzoate (at room temperature for 30 minutes × 2) and clearing with xylene (at room temperature for 30 minutes × 2). Then, the samples were immersed in a paraffin solution at 60°C (for 40 minutes × 3), embedded in paraffin and stored at 4°C.

### (2) Preparation or specimens by PFA-Paraffin method

The tissues used were obtained from patients with prostate cancer or benign prostatic hyperplasia who consented to use of their excised tissues for research purposes. The obtained samples were each trimmed to about 5 mm³ size and fixed in PFA fixative at 4°C for 16 to 24 hours. The fixed samples were subjected to serial dehydration with ethanol (70% ethanol at room temperature for 2 hours, 80% ethanol at room temperature for 1.5 hours, 90% ethanol at room temperature for 1.5 hours, 95% ethanol at room temperature for 1.5 hours, and 100% ethanol at room temperature for 2 hours × 3) and clearing with xylene (at room temperature for 1 hour × 2 and at room temperature for 1.5 hours × 1). Then, the samples were immersed in a paraffin solution at 60°C (for 1.5 hours × 1 and for 2 hours × 2), embedded in paraffin and stored at room temperature.

### (3) Preparation of specimens by NBF-Paraffin method

The tissues used were obtained from patients with prostate cancer or benign prostatic hyperplasia who consented to use of their excised tissues for research purposes. The obtained samples were each trimmed to about 5 mm³ size and fixed in 20% NBF fixative (20% formalin/0.1 M PB (pH 7.4)) at 4°C for 16 to 24 hours. The fixed samples were subjected to serial dehydration with ethanol (70% ethanol at room temperature for 2 hours, 80% ethanol at room temperature for 1.5 hours, 90% ethanol at room temperature for 1.5 hours, 95% ethanol at room temperature for 1.5 hours, and 100% ethanol at room temperature for 2 hours × 3) and clearing with xylene (at room temperature for 1 hour × 2 and at room temperature for 1.5 hours × 1). Then, the samples were immersed in a paraffin solution at 60°C (for 1.5 hours × 1 and for 2 hours × 2), embedded in paraffin and stored at room temperature.

### (4) Preparation of frozen specimens

The tissues used were obtained from patients with prostate cancer or benign prostatic hyperplasia who consented to use of their excised tissues for research purposes. The obtained samples were each trimmed to about 5 mm³ size, embedded in OCT compound (Sakura Finetek Japan Co., Ltd.) filled into plastic dishes (Cryomold^{®}, Sakura Finetek Japan Co., Ltd.), and then floated on the surface of hexane surrounded and cooled by dry ice and acetone to solidify the OCT compound by cooling. The frozen blocks thus prepared were transferred, together with the plastic dishes, to plastic containers containing hexane, and stored at -80°C.

### (5) Preparation of specimens from cultured human prostate cancer cell (LNCaP cell) tissues

Superimmunodeficient mice (NOG mice) were transplanted with LNCap cells (in vitro/in vivo cell line of human prostate cancer). The cells were collected from the mice at 54 to 67 days after transplantation, trimmed to about 5 mm³ size, and then processed according to the above four specimen preparation methods (PFA-AMeX-Paraffin method, PFA-Paraffin method, NBF-Paraffin method, fresh-frozen method) to prepare specimens for each method.

### Example 2: Observation of tissue morphology

For preparation and staining of thin sections from the paraffin blocks prepared by the PFA-AMeX-paraffin method, commercially available standard instruments and reagents were used. More specifically, paraffin sections of 4 µm thickness were prepared in a standard manner from the paraffin blocks, and each section was mounted on a foiled frame for an AS-LMD system (Leica Microsystems) and then dried. After deparaffinization with xylene (at room temperature for 15 seconds × 2) and rehydration with acetone (at room temperature for 15 seconds × 2), the sections were washed with RNase-free water and immersed in Mayer's hematoxylin solution (at room temperature for 10 seconds). After washing again with RNase-free water, the sections were immersed in an eosin solution (at room temperature for 10 seconds) and then dehydrated with ethanol (100% ethanol, at room temperature for 10 seconds). The sections stained with hematoxylin-eosin (HE) were dried and then observed for tissue morphology under a microscope attached to the AS-LMD system.

For preparation and staining of thin sections from the OCT-compound-embedded frozen tissue blocks prepared by the fresh-frozen method, standard instruments and reagents were used. More specifically, frozen sections of 6 µm thickness were prepared in a standard manner from the frozen blocks, and each section was mounted on a foiled frame for an AS-LMD system (Leica Microsystems). After fixation with methanol (at room temperature for 5 minutes), the sections were washed with RNase-free water and immersed in Mayer's hematoxylin solution (at room temperature for 30 seconds). After washing again with RNase-free water, the sections were immersed in an eosin solution (at room temperature for 30 seconds) and then dehydrated with ethanol (100% ethanol, at room temperature for 10 seconds). The HE-stained frozen sections were dried and then observed for tissue morphology under a microscope attached to the AS-LMD system.

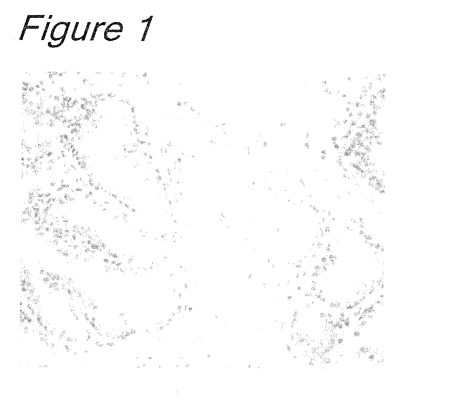

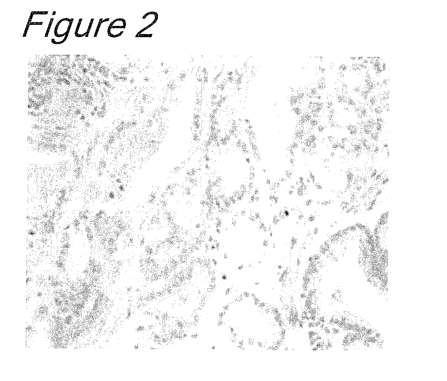

The frozen sections and the PFA-AMeX-processed paraffin sections were compared for tissue morphology of prostate cancer. In the frozen sections (Figure 1), it was difficult to distinguish the nuclear and nucleolar morphology of prostate epithelial cells and basal cells, indicating that there was a difficulty in distinguishing prostate cancer from the other lesions (PIN, hyperplastic lesions). In contrast, in the PFA-AMeX-processed paraffin sections (Figure 2), the above observations could be clearly confirmed, indicating that prostate cancer was distinguishable in these sections.

### Example 3: Extraction and quantification of total RNA from specimens prepared by four types of specimen preparation methods, as well as comparison of mRNA quality

Starting from the same tumor tissue of cultured human prostate cancer cells (LNCaP cells) transplanted into NOG mice, specimens were prepared according to the above four specimen preparation methods (fresh-frozen method, PFA-AMeX-Paraffin method, PFA-Paraffin method, NBF-Paraffin method). Total RNAs were extracted from thin sections of these specimens, followed by comparison of mRNA quality.

### (1) Extraction of total RNA from thin sections

Thin paraffin sections of the specimens prepared by the PFA-AMex-Paraffin method, the PFA-Paraffin method and the NBF-Paraffin method were respectively transferred to separate tubes (0.2 mL). After deparaffinization (xylene, at room temperature for 15 seconds × 3) and rehydration (100% ethanol, at room temperature for 15 seconds), the thin sections were dried. Then, 205 µL lysis buffer (20 mM Tris-HCl, 20 mM EDTA, 1% SDS, 500 µg/mL Proteinase K) was added, and the tubes containing the collected tissues were treated by heating (at 60°C for 16 hours and at 95°C for 10 minutes) and then cooled, followed by addition of 45 µL RNase-free water and 750 µL Trizol-LS (Invitrogen). After addition of Trizol-LS, the tubes were allowed to stand at room temperature for 5 minutes, and 200 µL Chloroform-Isoamyl alcohol solution was added thereto. The tubes were allowed to stand for 10 minutes at room temperature and then centrifuged. The supernatant was collected from each tube, to which 50 µL of 3 M NaOAc and 4 µL of 5 mg/mL glycogen were then added. After mixing, 500 µL isopropyl alcohol was added, and the mixture was allowed to stand for 10 minutes at room temperature and then centrifuged. The supernatant was removed and 1 mL of 75% ethanol was added to rinse the pellet. The pellet was further rinsed with 100% ethanol and then air-dried, followed by addition of 14 µL RNase-free water to dissolve the RNA pellet, which was then stored at - 80°C.

In the case of thin sections of the specimen prepared by the frozen method, total RNA was extracted with an RNeasy MinElute Kit (QIAGEN) and stored at -80°C.

### (2) Quantification of total RNA and test for mRNA quality

The amount of total RNA was quantified with a RiboGreen RNA Quantification Kit (Molecular Probes). After RNA quantification, 5 ng of the RNA was taken to another tube and treated with DNase to degrade contaminant DNA, and mRNA was then reverse-transcribed with oligo dT primers and reverse transcriptase (Superscript II RNase H-reverse Transcriptase; Invitrogen), followed by amplification with primer sets for amplifying the 5' side (amplification region: positions 1369-1521, 153 bp) and the 3' side (amplification region: positions 1600-1758, 159 bp) of human β-actin mRNA (X00351, 1761 bp) (whose cDNA sequence is shown in SEQ ID NO: 1). PCR products were quantified using SYBR Green for both 5' and 3' sides.

The primer set used for 5' side (1369-1521, 153 bp) PCR was shown below.

5'-ACAATGTGGCCGAGGACTTT-3' (1369-1388, 20 mer) (SEQ ID NO: 2) 5'-TGTGTGGACTTGGGAGAGGA-3' (1521-1502, 20 mer) (SEQ ID NO: 3) The primer set used for 3' side (1600-1758,159 bp) PCR was shown below.

5'-TTGTTTTATTTTGAATGATGAGCCTTCGT-3' (1600-1628, 29 mer) (SEQ ID NO: 4)
5'-GGTGTGCACTTTTATTCAACTGGTC-3' (1758-1734, 25 mer) (SEQ ID NO: 5)
Preliminary studies have confirmed that reliable Gene Chip data with >30% present-call on Affymetrix Human X3P arrays (% present-call is a value indicating what percentage of genes spotted on the array are determined as being "expressed") can be obtained for RNA samples which provide >1 units of 5' region amplification product and >15 units of 3' region amplification product, and whose 3' region/5' region PCR product ratio is <20. These values were used as criteria to determine the quality of each sample.

### (3) Comparison of mRNA quality in specimens prepared by four types of specimen preparation methods

Table 1 shows the results of mRNA quality compared among the specimens prepared by the four types of specimen preparation methods.

**[Table 1]**

| **Fresh Frozen** | | | | |
|---|---|---|---|---|
| Sample name | RNA conc (ng/ul) | β-actin 3' FW & RV (Set A)(unit) | β-actin 5' FW & RV (Set B)(unit) | Ratio (SetA/SetB) |
| M05-0458-01 | 27.14 | 44.690 | 58.520 | 0.76 |
| M05-0463-01 | 8.20 | 10.920 | 7.262 | 1.50 |
| M05-0464-01 | 29.09 | 26.940 | 19.170 | 1.41 |
| M06-0016-01 | 14.19 | 18.660 | 14.110 | 1.32 |
| M06-0017-01 | 7.72 | 4.072 | 3.367 | 1.21 |
| M06-0018-01 | 9.18 | 14.460 | 23.570 | 0.61 |
| average | 15.92 | 19.96 | 21.00 | 1.14 |

| **PFA-AMeX-paraffin** | | | | |
|---|---|---|---|---|
| Sample name | RNA conc (ng/ul) | β-actin 3' FW & RV (SetA)(unit) | β-actin 5' FW & RV (Set B)(unit) | Ratio (SetA/SetB) |
| M05-0458-21 | 74.31 | 7.325 | 2.570 | 2.85 |
| M05-0463-21 | 42.47 | 19.810 | 7.361 | 2.69 |
| M05-0464-21 | 54.18 | 24.050 | 9.525 | 2.52 |
| M06-0016-21 | 28.59 | 24.920 | 10.290 | 2.42 |
| M06-0017-21 | 75.62 | 35.050 | 11.650 | 3.01 |
| M06-0018-21 | 59.05 | 36.750 | 11.500 | 3.20 |
| average | 55.70 | 24.65 | 8.82 | 2.78 |

| **PFA-Paraffin** | | | | |
|---|---|---|---|---|
| Sample name | RNA conc (ng/ul) | β-actin 3' FW & RV (Set A)(unit) | β-actin 5' FW & RV (Set B)(unit) | Ratio (SetA/SetB) |
| M05-0458-11 | 49.35 | not amplified | 0.955 | N/A |
| M05-0463-11 | 37.48 | not amplified | 1.342 | N/A |
| M05-0464-11 | 14.47 | not amplified | 1.053 | N/A |
| M06-0016-11 | 53.93 | not amplified | 1.321 | N/A |
| M06-0017-11 | 157.46 | not amplified | 1.102 | N/A |
| M06-0018-11 | 86.78 | not amplified | 1.061 | N/A |
| average | 66.58 | not amplified | 1.14 | N/A |

| **NBF-Paraffin** | | | | |
|---|---|---|---|---|
| Sample name | RNA conc (ng/ul) | β-actin 3' FW & RV (Set A)(unit) | β-actin 5' FW & RV (Set B)(unit) | Ratio (SetA/SetB) |
| M05-0458-31 | 86.19 | 85.240 | 21.890 | 3.89 |
| M05-0463-31 | 76.18 | 31.760 | 7.252 | 4.38 |
| M05-0464-31 | 15.94 | 15.170 | 3.723 | 4.07 |
| M06-0016-31 | 21.82 | 21.340 | 4.751 | 4.49 |
| M06-0017-31 | 50.18 | 35.740 | 8.082 | 4.42 |
| M06-0018-31 | 17.20 | 38.110 | 8.801 | 4.33 |
| average | 44.59 | 37.89 | 9.08 | 4.27 |
| **Criteria** | | >15 | >1 | <20 |

The average value of the 3' region/5' region PCR product ratio was 1.14 in the frozen sections, 2.78 in the PFA-AMeX-Paraffin-processed samples, and 4.27 in the NBF-Paraffin-processed samples. In the PFA-Paraffin-processed samples, the PCR product ratio was not obtained because no PCR product was amplified for the 3' side due to degeneration. It should be noted that when PCR was also performed without reverse transcription in order to eliminate the possibility of genomic DNA contamination during PCR, samples obtained without reverse transcription provided no PCR amplification product, thus confirming that the PCR amplification products obtained were derived from the cDNAs produced by reverse transcription.

These results indicated that among the specimens prepared by the four types of specimen preparation methods, the quality of mRNA was highest in the frozen method, followed by the PFA-AMeX-Paraffin method and the NBF-Paraffin method, and lowest in the PFA-Paraffin method.

### Example 4: Comparison of mRNA quality in human prostate cancer tissue specimens prepared by PFA-A-MeX-Paraffin method and PFA-Paraffin method

To compare mRNA quality between human prostate cancer tissue specimens prepared by the PFA-AMeX-Paraffin method and the PFA-Paraffin method, thin sections were prepared from 79 PEA-AMeX-Paraffin specimens of human prostate cancer tissue and 69 PFA-Paraffin specimens of human prostate cancer tissue. These sections were treated in the same manner as shown in Example 3 to extract and quantify total RNA and to test mRNA quality. The results obtained are shown in Table 2.

**[Table 2]**

| Method used for specimen preparation | Total number of specimens tested | Number of specimens satisfying criteria |
|---|---|---|
| PFA-Paraffin method | 69 | 15 (21.7%) |
| PFA-AMex-Paraffin method | 79 | 53 (67.1%) |

In the human prostate cancer tissue specimens prepared by the PFA-Paraffin method, among all the specimens tested for mRNA quality, only 21.7% (15 of 69 specimens) were determined as having good mRNA quality. In contrast, in the human prostate cancer tissue specimens prepared by the PEA-AMeX-paraffin method, among all the samples tested for mRNA quality, 67.1% (53 of 79 samples) were determined as having good mRNA quality. Namely, the PFA-AMeX-paraffin tissue specimens showed a great improvement in mRNA quality, i.e., the percentage of specimens determined as having good mRNA quality was increased by 45.4% in these specimens when compared to the PFA-paraffin tissue specimens.

### Example 5: Tissue collection from thin sections with AS-LMD system, as well as extraction and quantification of total RNA from collected tissues, test for mRNA quality, and collection of Gene Chip data

### (1) Tissue collection with AS-LMD system

PFA-AMeX-paraffin specimens were prepared for human prostate tissue in the same manner as shown in Example 2. HE-stained thin sections were dried and then provided for sample collection with an AS-LMD system (Leica Microsystems) to collect target cells. More specifically, based on an image shown on a monitor of the AS-LMD system, prostate cancer lesions and prostatic hyperplasia lesions were distinguished and respectively collected to separate tubes (0.2 mL) using a laser. For total RNA extraction from the samples (collected tissues), 30 µL lysis buffer (20 mM Tris-HCl, 20 mM EDTA, 1% SDS, 500 µg/mL Proteinase K) had been added to each tube before sample collection, and 175 µL lysis buffer was further added after sample collection.

### (2) Extraction and quantification of total RNA from collected tissues and test for mRNA quality

The tissues collected by microdissection were treated in the same manner as shown in Example 3 to extract and quantify total RNA and to test mRNA quality.

### (3) Collection of Gene Chip data

Preliminary studies have confirmed that reliable Gene Chip data with >30% present-call on Affymetrix Human X3P arrays can be obtained for RNA samples which provide >1 units of 5' region amplification product and >15 units of 3' region amplification product, and whose 3' region/5' region PCR product ratio is <20 in the test for mRNA quality. These values were used as criteria to determine the quality of each sample. From the total RNAs of the samples each confirmed to satisfy the criteria and to contain mRNA of good quality, labeled cRNA probes were prepared according to standard procedures provided by Affymetrix, Inc., and gene expression data were obtained using Human X3P arrays (Affymetrix). Among the obtained gene expression data, those with >30% present-call were defined to be reliable data and provided for clustering analysis.

### Example 6: Analysis of gene expression data in prostate cancer tissue and prostatic hyperplasia tissue

Hierarchical clustering analysis (hierarchical clustering) was performed on the gene expression data in prostate cancer and prostatic hyperplasia tissues obtained in Example 5 to study the similarity of gene expression patterns. More specifically, gene expression data from 11 specimens of prostate cancer tissue and 42 specimens of prostatic hyperplasia tissue, each showing >30% present-call in Gene Chip gene expression, were each analyzed by the GC-RMA method to calculate a signal value (i.e., a value indicating the expression level of a gene), which was then converted into a logarithmic value and mean-centered (averaged, by which the starting point of plotting was shifted to the center of the data set, i.e., the mean of each variable was zero), followed by hierarchical clustering. For hierarchical clustering, the pvclust R package was used (R is a free software for statistical calculation and graphics).

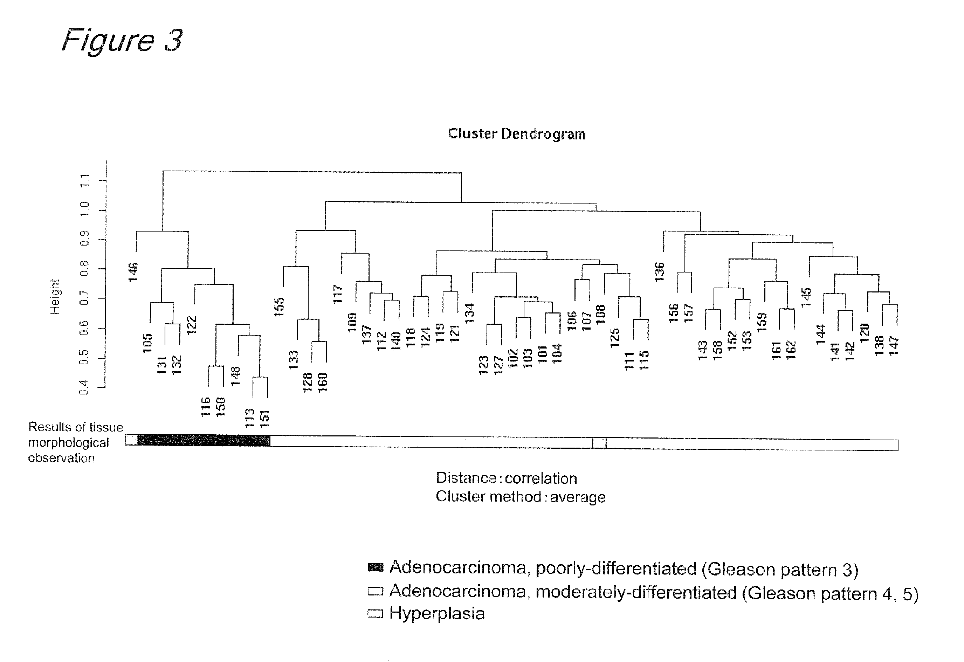

The results of clustering analysis are shown in Figure 3. As shown in the tree diagram (dendrogram) of Figure 3, prostate cancer specimens and prostatic hyperplasia specimens showed mutually different gene expression patterns, so that the prostate cancer specimen group and the prostatic hyperplasia specimen group were distinguished as separate clusters. On the other hand, there was a high similarity in gene expression pattern between prostate cancer specimens or between prostatic hyperplasia specimens.

Further, among the prostate cancer specimens, those determined by morphological observation as being poorly-differentiated adenocarcinoma or moderately-differentiated adenocarcinoma were found to show a higher similarity in gene expression pattern between poorly-differentiated adenocarcinoma specimens or between moderately-differentiated adenocarcinoma specimens than between poorly-differentiated and moderately-differentiated adenocarcinoma specimens, and specimens determined by morphological observation as being poorly-differentiated were found to form a cluster earlier than others. This result indicated that gene expression patterns faithfully reflecting the results of morphological observation were obtained. Unlike the PFA-AMeX-Paraffin prostate tissue specimens, even when the same experiment and data analysis are performed with frozen prostate tissue specimens, the same result showing that gene expression patterns faithfully reflect the results of tissue morphological observation has not been obtained.

### Example 7: Tissue morphology and RNA quality in SNAP-Frozen samples processed by PFA-AMeX method

In standard tissue banks, most tissue samples are frozen for storage (SNAP-Frozen samples), and these samples have been used primarily for extraction of nucleic acids and/or proteins. As to tissue morphological observation, although it is generally possible to use SNAP-Frozen samples for preparation of frozen sections, they have a difficulty in the maintenance of tissue morphology when compared to ordinary paraffin sections. For this reason, specimens were prepared for SNAP-Frozen samples (frozen tissues) by the PFA-AMeX-Paraffin method, and these specimens were evaluated for the maintenance of tissue morphology and for mRNA quality.

### (1) Preparation of PFA-AMeX-Paraffin specimens from SNAP-Frozen tissues

The frozen tissues (SNAP-Frozen tissues) used were obtained from patients with prostate cancer or benign prostatic hyperplasia who consented to use of their excised tissues for research purposes. The SNAP-Frozen samples were each trimmed to about 5 mm³ size and processed by the PFA-AMeX-Paraffin method as shown in Example 1 to prepare specimens.

### (2) Comparison of tissue morphology

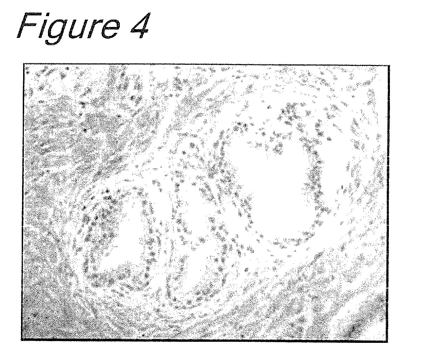

Although thin paraffin sections of the specimens prepared from the SNAP-Frozen (frozen) samples by the PFA-AMeX-Paraffin method showed a slight nuclear vacuolation in morphological observation after HE staining, it was possible to distinguish tissue morphology with substantially the same accuracy as in thin paraffin sections of PFA-AMeX-Paraffin specimens prepared from unfrozen tissues (Figure 4).

### (3) Test for mRNA quality

The specimens prepared from the SNAP-Frozen (frozen) samples by the PFA-AMeX-Paraffin method were treated in the same manner as shown in Example 4 to extract and quantify total RNA and to test mRNA quality. The results obtained are shown in Table 3.

**[Table 3]**

| | Total number of specimens tested | Number of specimens satisfying criteria |
|---|---|---|
| SNAP-Frozen PFA-AMex method | 10 | 7 (70.0%) |

Among the 10 specimens tested for mRNA quality, 7 specimens (70.0%) were determined as being mRNA of good quality. In the PFA-AMeX-Paraffin specimens prepared from the unfrozen tissue samples, 67.1% (53 of 79 specimens) were determined as being mRNA of good quality (Example 4, Table 2). In view of this finding, the mRNAs maintained in the PFA-AMeX-Paraffin specimens prepared from the SNAP-Frozen (frozen) samples were confirmed to be of substantially the same quality as that of the specimens prepared from the unfrozen samples.

## Claims

1. A method for specimen preparation from various frozen or unfrozen organs or tissues (excluding hard tissues) of the whole body, which comprises the following steps:
1) fixing a target organ or tissue with PFA fixative; and
2) embedding the same in paraffin by the AMeX method.

2. The method according to claim 1, wherein fixation with the PFA fixative is accomplished by immersion.

3. The method according to claim 1 or 2, which further comprises the step of preparing thin sections after paraffin embedding by the AMeX method.

4. The method according to claim 3, which further comprises the step of deparaffinizing and rehydrating the resulting thin sections.

5. The method according to claim 4, which further comprises the step of tissue staining, immunohistochemical staining or enzyme histochemical staining.

6. The method according to any one of claims 1 to 5, which is a method for preparing a prostate tissue specimen.

7. The method according to any one of claims 1 to 5, which is a method for preparing a prostate cancer tissue specimen.

8. Specimens of various organs or tissues (excluding hard tissues) of the whole body, which are obtainable by the method according to any one of claims 1 to 5.

9. The specimen according to claim 8, which is a prostate tissue specimen.

10. The specimen according to claim 8, which is a prostate cancer tissue specimen.

11. A method for testing the quality of mRNA in cells within an organ or tissue specimen, which comprises the following steps:
1) collecting desired cells from an organ or tissue specimen obtainable by the method according to any one of claims 1 to 7;
2) extracting total RNA from the collected desired cells;
3) synthesizing cDNA from the extracted total RNA by reverse transcription reaction;
4) using the synthesized cDNA as a template to amplify the 5- and 3-terminal regions of β-actin by PCR; and
5) calculating the ratio of amplification products between 5- and 3-terminal regions of β-actin, whereby cells whose 3-terminal region/5-terminal region PCR product ratio is below a given value are determined as having good mRNA quality.

12. The method according to claim 11, wherein the desired cells are collected by microdissection from the organ or tissue specimen.

13. A method for preparing a DNA microarray sample, which comprises the following steps:
1) collecting desired cells from an organ or tissue specimen obtainable by the method according to any one of claims 1 to 7;
2) testing the quality of mRNA in the collected desired cells in the following manner:
a) extracting total RNA from the collected desired cells;
b) synthesizing cDNA from the extracted total RNA by reverse transcription reaction;
c) using the synthesized cDNA as a template to amplify the 5- and 3-terminal regions of β-actin by PCR; and
d) calculating the ratio of amplification products between 5- and 3-terminal regions of β-actin, whereby cells whose 3-terminal region/5-terminal region PCR product ratio is below a given value are determined as having good mRNA quality; and
3) extracting total RNA from the cells determined in step 2) as having good mRNA. quality and using the same to synthesize cDNA, from which cRNA is further synthesized.

14. A method for performing hierarchical clustering analysis in a DNA microarray, which uses the sample obtained by the method according to claim 13.
